# FASCICULE DE BREVET EUROPEEN

(11) **EP 1 117 453 B1**
(45) Date de publication et mention de la délivrance du brevet: **17.11.2004**
(21) Numéro de dépôt: 99946267.4
(22) Date de dépôt: 30.09.1999
(51) Int. Cl.: A61M 5/28

(54) **DISPOSITIF D'INJECTION A USAGE UNIQUE DESTINE A ETRE PRE-REMPLI**
VORGEFÜLLTE INJEKTIONSVORRICHTUNG FÜR EINMALVERWENDUNG
DISPOSABLE INJECTION DEVICE DESIGNED TO BE PRE-FILLED

(30) Priorité: 01.10.1998 FR 9812309
(43) Date de publication de la demande: 25.07.2001
(73) Titulaire: Sanofi-Aventis, 75013 Paris (FR)
(72) Inventeur: Brunel, Marc, F-31000 Toulouse (FR)
(74) Mandataire: Cabinet BARRE LAFORGUE & associés
(86) Numéro de dépôt international: PCT/FR1999/002326
(87) Numéro de publication internationale: WO 2000/020057

(56) Documents cités:
- EP-A- 0 112 574
- US-A- 4 226 236
- US-A- 4 657 534

## Description

L'invention concerne un dispositif d'injection du type à usage unique conçu pour être pré-rempli d'une dose de liquide, notamment médicamenteux, à injecter.

Les dispositifs d'injection à usage unique destinés à être pré-remplis comportent un corps de seringue, soit doté d'une embase dans laquelle est scellée une aiguille protégée par un capuchon de protection, soit comportant un raccord conique mâle à verrouillage permettant d'adapter sur ledit corps de seringue un raccord conique femelle à verrouillage portant une aiguille d'injection protégée par un capuchon de protection, lesdits raccords coniques définissant un assemblage communément connu sous l'appellation "assemblage conique LÜER".

Les dispositifs d'injection les plus classiques dits à "aiguille humide" sont du type doté d'un corps de seringue comportant une embase dans laquelle est scellée une aiguille obturée au moyen d'un capuchon de protection en élastomère doté d'un alésage borgne interne ménagé dans le fond dudit capuchon, à l'intérieur duquel vient se loger en force l'extrémité de ladite aiguille de façon à garantir l'étanchéité du dispositif d'injection avant injection.

Le premier inconvénient de tels dispositifs d'injection réside dans le fait qu'ils imposent, lors de la mise en place du capuchon de protection, de centrer parfaitement l'aiguille d'injection par rapport à l'alésage dudit capuchon. Or, dans la pratique, ce centrage s'avère parfois approximatif, de sorte que le montage du capuchon conduit fréquemment à détériorer ledit capuchon ou l'aiguille, avec pour conséquence des rebuts non négligeables de fabrication.

De plus, selon ce principe, la qualité de l'aiguille (affûtage, siliconage) se trouve systématiquement affectée du fait des frottements de la pointe de ladite aiguille contre la paroi interne de l'alésage borgne du capuchon lors du montage en force de cette dernière.

Enfin, le liquide renfermé dans ces dispositifs d'injection se trouve obligatoirement au contact des matériaux constituant l'aiguille d'injection et le capuchon de protection qui peuvent, pour certains types de liquide, affecter la conservation de ces derniers.

Pour pallier ces inconvénients, il a été conçu de multiples dispositifs d'injection dits "à aiguille sèche", pour lesquels l'aiguille d'injection se trouve isolée du liquide renfermé dans le corps de seringue jusqu'au moment de l'injection.

Un premier type de dispositif d'injection "à aiguille sèche" est celui utilisé couramment dans le domaine dentaire et comporte un flacon renfermant le liquide à injecter et obturé par une membrane, et une aiguille bi-pointe apte à être déplacée axialement par rapport audit flacon, de façon à venir percer la membrane au moment de l'injection. De tels dispositifs d'injection sont notamment décrits dans les brevets DE-847473, FR-2347055, US-4639250, EP-602883, DE-2008751, DE-1909794.

Les inconvénients de ce type de dispositif d'injection sont de deux ordres. En effet, et en premier lieu, le fait de nécessiter une aiguille bi-pointe conduit à un surcoût concernant le prix de revient de ces dispositifs d'injection, résultant d'une part du coût en lui-même de ladite aiguille, et d'autre part, de l'obligation d'effectuer deux opérations d'affûtage en lieu et place de la seule opération d'affûtage requise pour une aiguille classique. De plus, il arrive fréquemment, notamment pour des petits diamètres d'aiguille, que l'on se trouve confrontés à des problèmes de carottage conduisant à l'inclusion de particules de membrane à l'intérieur de la lumière de l'aiguille, qui soit obturent cette lumière, soit sont injectés avec le liquide.

Un deuxième type de dispositif d'injection à "aiguille sèche", comporte un corps de seringue logeant deux bouchons délimitant la chambre renfermant le liquide, et sur lequel est sertie une embase soit portant une aiguille, soit du type raccord conique mâle, ledit corps de seringue possédant, en outre, un compartiment doté d'un conduit de communication avec l'aiguille d'injection, ménagé de façon à être mis en relation avec la chambre uniquement après déplacement axial des pistons.

De tels dispositifs d'injection, notamment décrits dans les brevets FR-2412320, FR-2208684, EP-191508, EP-588148 et EP-720857, permettent de pallier les inconvénients des dispositifs d'injection ci-dessus décrits. Toutefois, ils présentent également eux-mêmes deux inconvénients. En effet, et en premier lieu, l'opération de sertissage de l'embase sur le corps de seringue s'avère délicate et exige une attention particulière en vue de garantir une parfaite étanchéité entre ladite embase et ledit corps de seringue. De plus et surtout, de tels dispositifs d'injection peuvent être sujets à des écoulements accidentels du liquide renfermé dans la chambre, résultant par exemple d'une dilatation du volume de gaz renfermé dans ladite chambre, ou d'une dépressurisation notamment lors d'un transport aérien, qui conduisent à provoquer le déplacement axial du bouchon d'accès au compartiment d'évacuation du liquide.

Un troisième type de dispositif d'injection à "aiguille sèche" décrit notamment dans les brevets EP-150681, EP-111796, FR-2330413, US-4226236 et WO-8404252, permet de pallier l'ensemble des inconvénients sus-évoqués. A cet effet, ces dispositifs d'injection comportent, d'une part, un corps de seringue doté d'une chambre obturée par un bouchon en caoutchouc percé d'un alésage longitudinal traversant, et d'autre part une embase soit portant une aiguille soit du type raccord conique mâle mobile axialement à l'intérieur de l'alésage dudit bouchon, et dotée de conduits ménagés de façon à mettre en communication l'aiguille d'injection et la chambre lors d'un déplacement axial de ladite embase tendant à l'enfoncer dans le bouchon.

La présente invention vise un dispositif d'injection du type à assemblage conique à verrouillage de conception similaire à celle des dispositifs d'injection du troisième type décrit ci-dessus, et a pour principal objectif de fournir un dispositif d'injection ergonome alliant les avantages de ces dispositifs d'injection (étanchéité, garantie contre les risques d'écoulement accidentel...) et dont l'activation en vue d'une injection s'opère de façon très simple par un geste très naturel.

Ainsi, l'invention vise plus particulièrement un dispositif d'injection du type décrit dans US-4226236, et comprenant un corps de seringue délimitant une chambre destinée à être remplie d'un liquide, notamment médicamenteux, et un organe d'obturation de la chambre et de distribution du liquide, comportant un embout d'obturation de ladite chambre prolongé d'un raccord conique mâle de verrouillage percé d'un conduit de distribution du liquide :
- l'organe d'obturation et de distribution étant mobile axialement entre une position, dite position d'obturation, où le conduit de distribution est isolé de la chambre du corps de seringue, et une position, dite position d'injection, où ledit conduit de distribution communique avec ladite chambre,
- l'organe d'obturation et de distribution comprenant un collet intermédiaire disposé entre l'embout d'obturation et le raccord conique mâle, et agencé pour s'étendre dans un prolongement du corps de seringue,
- le corps de seringue comprenant, comme prolongement, une bague dotée d'une paroi frontale percée d'un orifice axial pour le passage du raccord conique mâle, ladite bague délimitant deux compartiments internes, supérieur et inférieur, juxtaposés axialement, chacun de forme conjuguée du collet intermédiaire de l'organe d'obturation et de distribution, apte à loger ledit collet intermédiaire, lesdits compartiments étant séparés par des organes de butée axiale aptes à autoriser un déplacement axial du collet intermédiaire d'un compartiment vers l'autre compartiment.

Selon l'invention, la bague de ce dispositif d'injection comporte un prolongement par rapport à la paroi frontale, consistant en un manchon fileté intérieurement agencé pour loger le raccord conique mâle et faire office d'écrou fixe pour l'assemblage d'un raccord conique femelle à verrouillage portant une aiguille d'injection.

L'activation d'un tel dispositif d'injection, en vue de l'injection, est donc obtenue automatiquement lors de l'assemblage par vissage d'un raccord conique femelle sur le corps de seringue, qui entraîne un déplacement axial de l'organe d'obturation et de distribution du compartiment supérieur de la bague vers le compartiment inférieur de ladite bague, conduisant à mettre en communication l'aiguille et la chambre dudit corps de seringue.

Cette activation résulte donc du simple montage sur le dispositif d'injection, d'un raccord conique femelle constitutif d'un assemblage conique classique par exemple de type "LÜER".

De plus, le déplacement axial de l'organe d'obturation et de distribution est irréversible, du fait que tout déplacement ultérieur inverse s'avère par la suite interdit.

Selon un mode de réalisation avantageux de l'invention, la bague présente intérieurement un col annulaire formant le sommet de deux divergents longitudinaux et constituant les moyens de butée axiale de séparation des compartiments supérieur et inférieur, le collet intermédiaire de l'organe d'obturation et de distribution présentant une forme et des dimensions conjuguées du compartiment supérieur de ladite bague.

Par ailleurs, le dispositif d'injection selon l'invention comprend avantageusement un bouchon d'obturation de dimensions adaptées pour être inséré dans le corps de seringue, percé d'un alésage traversant débouchant dans ledit corps de seringue au niveau d'un lamage.

De plus, dans ce cas, l'organe d'obturation et de distribution présente des dimensions adaptées pour être inséré dans l'alésage du bouchon d'obturation, et comprend un conduit de distribution comportant une branche transversale ménagée de façon à être positionnée en retrait du lamage dudit bouchon d'obturation, dans la position d'obturation de l'organe d'obturation et de distribution, et pour s'étendre dans ledit lamage dans la position d'injection dudit organe.

D'autres caractéristiques, buts et avantages de l'invention ressortiront de la description détaillée qui suit en référence aux dessins annexés qui en représentent à titre d'exemple non limitatif un mode de réalisation préférentiel. Sur ces dessins :
- la figure 1 est une coupe axiale longitudinale d'un dispositif d'injection conforme à l'invention,
- la figure 2 est une coupe axiale longitudinale de ce dispositif d'injection après assemblage d'un raccord conique femelle portant une aiguille d'injection protégée par un capuchon de protection,

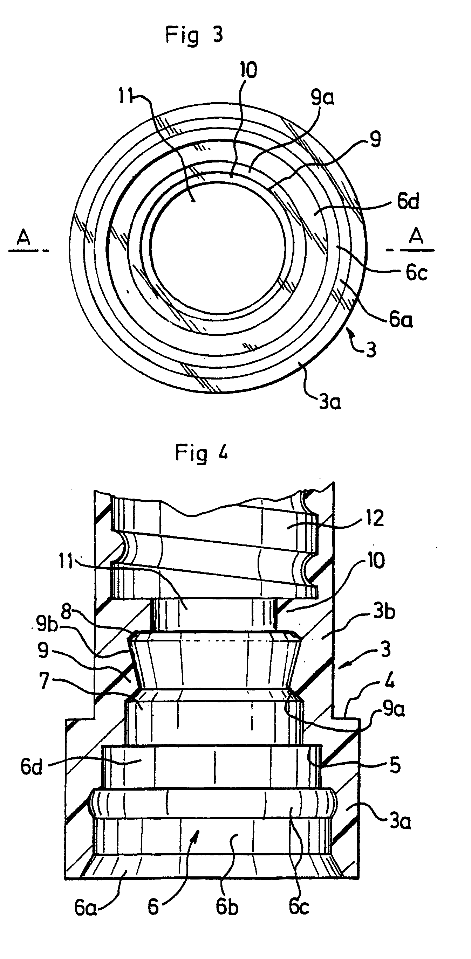

- la figure 3 est une vue frontale de dessous de la bague du corps de seringue de ce dispositif d'injection,
- la figure 4 est une coupe longitudinale par un plan axial A de cette bague,

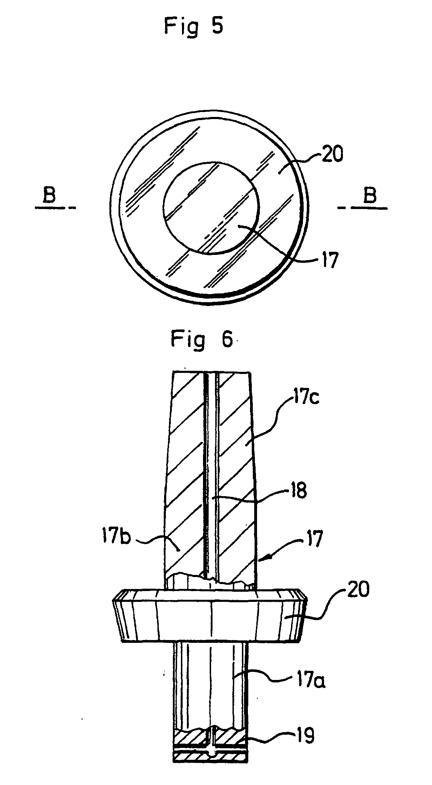

- la figure 5 est une vue frontale de dessous de l'organe d'obturation et de distribution de ce dispositif d'injection,
- et la figure 6 est une vue longitudinale partiellement en coupe par un plan axial B de cet organe d'obturation et de distribution.

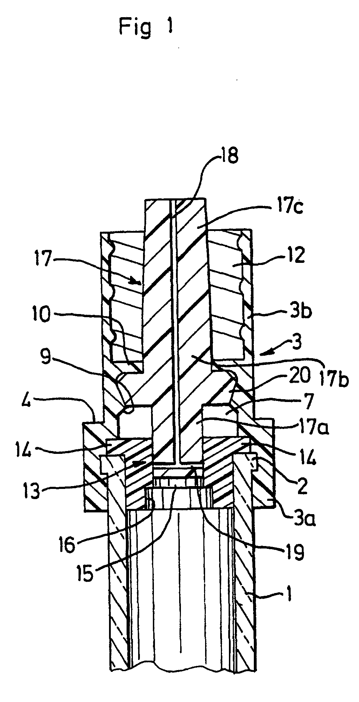

Le dispositif d'injection représenté à la figure 1 est du type à usage unique, c'est-à-dire destiné à être rempli d'un liquide à injecter. Il est à noter que bien que le dispositif d'injection tel que représenté ne possède pas d'étui protecteur destiné à permettre de protéger l'aiguille sans risque de piqûre, après injection, il peut être équipé, de façon connue en soi, de tout étui protecteur de type classique remplissant cette fonction.

Ce dispositif d'injection comprend, en premier lieu, un corps de seringue classique comportant un tube cylindrique 1 doté d'une collerette externe 2 au niveau d'une de ses extrémités.

Ce dispositif d'injection comprend, en outre, une bague 3 adaptée pour venir s'adapter sur le tronçon d'extrémité du tube cylindrique 1.

Cette bague 3 se compose longitudinalement de deux manchons cylindriques 3a, 3b de diamètres externe et interne différents, séparés par des épaulements respectivement externe 4 et interne 5.

Le premier manchon 3a, de plus grands diamètres, est adapté pour venir s'emmancher sur sa plus grande longueur sur le tronçon d'extrémité du tube cylindrique 1 du corps de seringue. Intérieurement, ce manchon 3a présente un alésage 6 divisé longitudinalement en quatre tronçons consistant successivement en :
- un premier tronçon d'extrémité 6a destiné à être présenté en regard de l'extrémité du corps de seringue lors du montage de la bague 3, et présentant une forme tronconique définissant une rampe apte à faciliter ce montage,
- un premier tronçon intermédiaire 6b de forme cylindrique de diamètre interne conjugué du diamètre externe du tube cylindrique 1,
- un deuxième tronçon intermédiaire 6c de forme torique adapté pour venir s'encliqueter sur la collerette 2 du tube cylindrique 1,
- et un quatrième tronçon 6d, de jonction avec le deuxième manchon 3b, de forme cylindrique de diamètre interne légèrement inférieur à celui du premier tronçon intermédiaire 6b.

Le second manchon 3b est lui-même divisé intérieurement en deux tronçons longitudinaux séparés par une paroi transversale intermédiaire 10 percée d'un orifice axial 11.

Le premier de ces tronçons, inférieur, séparé du tronçon de jonction 6d du premier manchon 3a par l'épaulement interne 5, est lui-même divisé longitudinalement en deux compartiments superposés de même hauteur 7, 8, par un col annulaire 9 formant le sommet de deux divergents longitudinaux 9a, 9b :
- un compartiment inférieur 7 présentant dans le prolongement inférieur du premier divergent 9a, un tronçon cylindrique de jonction avec le tronçon 6d du premier manchon 3a,
- un compartiment supérieur 8 délimité latéralement par le deuxième divergent 9b, et relié à la paroi intermédiaire 10 par un divergent parallèle au premier divergent 9a, et donc de section décroissant en direction de ladite paroi intermédiaire.

Le second tronçon, supérieur, du second manchon 3b constitue, quant à lui, un collier 12 de raccord mâle à verrouillage pour assemblage conique de type "LÜER". Un tel collier étant de type classique et notamment de forme et dimensions définies par des normes ne sera donc pas décrit plus en détail dans la présente demande.

Le dispositif d'injection comporte, en outre, des moyens d'obturation de l'extrémité du tube cylindrique 1 du corps de seringue, aptes à permettre d'autoriser l'injection du liquide renfermé dans ledit tube.

Ces moyens d'obturation comprennent, en premier lieu, un bouchon cylindrique 13 apte à être inséré dans le tube cylindrique 1 et doté d'une collerette cylindrique 14 de butée sur l'extrémité dudit tube.

Ce bouchon 13 est, en outre, percé d'un alésage cylindrique traversant 15 débouchant dans un lamage 16 ménagé dans la face frontale dudit bouchon opposée à la collerette 14.

Ces moyens d'obturation comprennent, en outre, un organe d'obturation et de distribution 17 adapté pour venir se loger dans l'alésage 15 du bouchon 13, et s'étendre à l'intérieur de la bague 3.

Cet organe d'obturation et de distribution 17 se présente sous la forme générale d'un fût comportant un collet intermédiaire 20 de forme et dimensions conjuguées de celles du compartiment supérieur 8 du second manchon 3b de la bague 3.

La portion 17a de ce fût 17 s'étendant dans le prolongement inférieur du collet 20, présente une forme cylindrique de diamètre adapté pour pénétrer dans l'alésage 15 du bouchon 13.

La portion de ce fût 17 s'étendant dans le prolongement supérieur du collet 20 comporte, quant à elle, un premier tronçon cylindrique 17b de diamètre supérieur à celui de la portion inférieure 17a dudit fût, prolongé par un tronçon tronconique 17c de forme et dimensions adaptées pour constituer l'embout conique du raccord mâle à verrouillage d'un "assemblage conique LÜER".

L'organe d'obturation et de distribution 17 est, par ailleurs, percé axialement, sur sa plus grande longueur à partir de sa face frontale supérieure, d'un conduit longitudinal 18 débouchant dans un conduit transversal traversant 19 ménagé dans la portion 17a du fût, à faible distance de la face frontale inférieure dudit fût.

Le dispositif d'injection comprend, par ailleurs, un raccord conique femelle à verrouillage 30 pour assemblage conique de type "LÜER". Ce raccord conique 30, de type connu en soi, comporte classiquement une embase conique 23 dans laquelle est sertie une aiguille d'injection 24, et dans le prolongement inférieur de ladite embase, un manchon conique 21 conjugué de l'embout conique 17c du raccord mâle, doté d'ailettes 22 aptes à permettre le vissage dudit raccord femelle dans le collier 12.

Ce dispositif d'injection comprend, enfin, un capuchon de protection 25 de forme adaptée pour venir coiffer l'embase 23 du raccord conique femelle 30 et loger l'aiguille d'injection 24, ledit capuchon de protection comportant, de façon classique, une collerette externe 26 de butée sur le collier 12.

L'assemblage d'un tel dispositif d'injection consiste simplement, en premier lieu, à introduire l'organe d'obturation et de distribution 17 à l'intérieur de la bague 3 jusqu'à amener le collet intermédiaire 20 dans le compartiment supérieur 8 de ladite bague.

Le bouchon 13 est ensuite également mis en place dans la bague 3, dans une position où la collerette 14 dudit bouchon vient se loger dans le quatrième tronçon 6d du premier manchon 3a de ladite bague.

Dans cette position du bouchon 13, et tel que représenté à la figure 1, le conduit transversal 19 de l'organe d'obturation et de distribution 17 se trouve positionné dans l'alésage 15 dudit bouchon, en retrait par rapport au lamage 16 de ce dernier. De plus, ce bouchon 13 obture frontalement le compartiment inférieur 7 de la bague 3, de façon à limiter le déplacement axial de l'organe d'obturation et de distribution 17.

La dernière opération consiste enfin à présenter le corps de seringue en regard des éléments pré-assemblés ci-dessus décrits, et à emmancher ledit corps de seringue à l'intérieur de la bague 3 jusqu'à amener la collerette 2 du tube cylindrique 1 à se clipser dans le tronçon intermédiaire torique 6c du premier manchon 3a de ladite bague.

Après remplissage du corps de seringue et obturation de ce dernier par un corps de piston de type classique, le dispositif d'injection peut alors être manipulé, stocké, ..., sans risque d'écoulement de liquide ni de contamination de ce liquide.

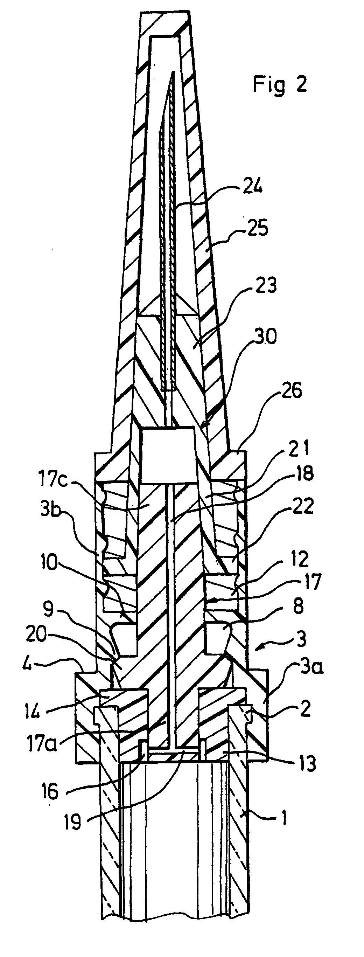

En vue d'une injection, et tel que représenté à la figure 2, il suffit à l'utilisateur d'assembler par vissage le raccord conique femelle 30 à verrouillage.

Lors de cet assemblage, l'organe d'obturation et de distribution 17 est amené automatiquement à se déplacer axialement vers une position d'injection où, d'une part le collet intermédiaire 20 vient se loger dans le compartiment inférieur 7 de la bague 3, et d'autre part, le conduit transversal 19 débouche dans le lamage 16 du bouchon 13 autorisant l'écoulement et l'injection du liquide.

## Revendications

1. Dispositif d'injection comprenant un corps de seringue (1, 13) délimitant une chambre destinée à être remplie d'un liquide, notamment médicamenteux, et un organe (17) d'obturation de la chambre et de distribution du liquide, comportant un embout (17a) d'obturation de ladite chambre, prolongé d'un raccord conique mâle (17c) de verrouillage percé d'un conduit (18, 19) de distribution du liquide :
- l'organe d'obturation et de distribution (17) étant mobile axialement entre une position, dite position d'obturation, où le conduit de distribution (18, 19) est isolé de la chambre du corps de seringue (1, 13), et une position, dite position d'injection, où ledit conduit de communication communique avec ladite chambre,
- l'organe d'obturation et de distribution (17) comprenant un collet intermédiaire (20) disposé entre l'embout d'obturation (17a) et le raccord conique mâle (17c), et agencé pour s'étendre dans un prolongement (3) du corps de seringue (1, 13),
- le corps de seringue (1, 13) comprenant, comme prolongement, une bague (3) dotée d'une paroi frontale (10) percée d'un orifice (11) pour le passage du raccord conique mâle (17c), ladite bague délimitant deux compartiments internes, supérieur (8) et inférieur (7), juxtaposés axialement, chacun de forme conjuguée du collet intermédiaire (20) de l'organe d'obturation et de distribution (17), apte à loger ledit collet intermédiaire, lesdits compartiments étant séparés par des organes de butée axiale (9) aptes à autoriser un déplacement axial du collet intermédiaire (20) d'un compartiment (7, 8) vers l'autre compartiment (8, 7),
ledit dispositif d'injection étant **caractérisé en ce que** la bague (3) comporte un prolongement par rapport à la paroi frontale (10), consistant en un manchon (12) fileté intérieurement agencé pour loger le raccord conique (17c) mâle et à faire office d'écrou fixe pour l'assemblage d'un raccord conique femelle (30) à verrouillage portant une aiguille d'injection (24).

2. Dispositif d'injection selon la revendication 1, **caractérisé en ce que** la bague (3) présente intérieurement un col annulaire (9) formant le sommet de deux divergents longitudinaux (9a, 9b) et constituant les moyens de butée axiale de séparation des compartiments supérieur (8) et inférieur (7), le collet intermédiaire (20) de l'organe d'obturation et de distribution (17) présentant une forme et des dimensions conjuguées du compartiment supérieur (8) de ladite bague.

3. Dispositif d'injection selon l'une des revendications 1 ou 2, **caractérisé en ce que** :
- il comprend un bouchon d'obturation (13) de dimensions adaptées pour être inséré dans le corps de seringue, percé d'un alésage traversant (15) débouchant dans ledit corps de seringue au niveau d'un lamage (16),
- l'organe d'obturation et de distribution (17) présente des dimensions adaptées pour être inséré dans l'alésage (15) du bouchon d'obturation (13) et comprend un conduit de distribution (18, 19) comportant une branche d'extrémité transversale (19) ménagée de façon à être positionnée en retrait du lamage (16) dudit bouchon d'obturation (13), dans la position d'obturation de l'organe d'obturation et de distribution (17), et pour s'étendre dans ledit lamage dans la position d'injection dudit organe.

## Patentansprüche

1. Injektionsvorrichtung, umfassend einen Spritzenkörper (1, 13), der eine mit einer Flüssigkeit, insbesondere einem Medikament, zu füllende Kammer begrenzt, und einen Mechanismus (17) zum Verschließen der Kammer und zum Ausgeben der Flüssigkeit, der ein Ansatzstück (17a) zum Verschließen der genannten Kammer umfasst, verlängert durch ein konisches Sperrsteckverbindungsstück (17c), das von einem Kanal (18, 19) zum Ausgeben der Flüssigkeit durchbohrt ist:
- wobei der Verschluss- und Ausgabemechanismus (17) axial zwischen einer Position, Verschlussposition genannt, in der der Ausgabekanal (18, 19) von der Kammer des Spritzenkörpers (1, 13) isoliert ist, und einer Position, Injektionsposition genannt, beweglich ist, in der der genannte Verbindungskanal mit der genannten Kammer in Verbindung ist,
- wobei der Verschluss- und Ausgabemechanismus (17) eine Zwischenhülse (20) aufweist, die zwischen dem Verschlussansatzstück (17a) und dem konischen Sperrsteckverbindungsstück (17c) angeordnet und so gestaltet ist, dass sie in einer Verlängerung (3) des Spritzenkörpers (1, 13) verläuft,
- wobei der Spritzenkörper (1, 13) als Verlängerung einen Ring (3) umfasst, der mit einer Frontwand (10) versehen ist, die von einer Öffnung (11) für die Passage des konischen Sperrsteckverbindungsstücks (17c) durchbohrt ist, wobei der genannte Ring zwei Innenkammern, eine obere (8) und eine untere (7) begrenzt, die axial nebeneinander liegen, jeweils mit einer Form, die zur Zwischenhülse (20) des Verschluss- und Ausgabemechanismus (17) passt, und die die genannte Zwischenhülse aufnehmen kann, wobei die genannten Kammern durch axiale Anschlagelemente (9) getrennt sind, die so gestaltet sind, dass sie eine axiale Verschiebung der Zwischenhülse (20) der einen Kammer (7, 8) in Richtung auf die andere Kammer (8, 7) zulassen,
wobei die genannte Injektionsvorrichtung **dadurch gekennzeichnet ist, dass** der Ring (3) eine Verlängerung in Bezug auf die Frontwand (10) aufweist, bestehend aus einem Gewindestutzen (12), der innen so gestaltet ist, dass er das konische Steckverbindungsstück (17c) aufnimmt, und der als feste Schraubenmutter für die Montage eines konischen Aufnahmesteckverbindungsstückes (30) dient, das eine Injektionsnadel (24) trägt.

2. Injektionsvorrichtung nach Anspruch 1, **dadurch gekennzeichnet, dass** der Ring (3) innen einen ringförmigen Vorsprung (9) aufweist, der der Scheitelpunkt von zwei Längsdivergierenden (9a, 9b) ist und die axialen Anschläge zum Trennen der oberen (8) und der unteren (7) Kammer bildet, wobei die Zwischenhülse (20) des Verschluss- und Ausgabemechanismus (17) so gestaltet und dimensioniert ist, dass sie zur oberen Kammer (8) des genannten Rings passt.

3. Injektionsvorrichtung nach Anspruch 1 oder 2, **dadurch gekennzeichnet, dass**:
- sie eine Verschlusskappe (13) mit solchen Abmessungen umfasst, dass sie in den Spritzenkörper eingeführt werden kann, der von einer Durchgangsbohrung (15) durchbohrt ist, die in dem genannten Spritzenkörper in der Höhe einer Senkung (16) mündet,
- der Verschluss- und Ausgabemechanismus (17) solche Abmessungen hat, dass er in die Bohrung (15) der Verschlusskappe (13) eingeführt werden kann, und der einen Ausgabekanal (18, 19) umfasst, der einen transversalen Endzweig (19) aufweist, der so gestaltet ist, dass er in der Verschlussposition des Verschluss- und Ausgabemechanismus (17) so in die Senkung (16) der genannten Verschlusskappe (13) eingelassen werden kann, dass er in der genannten Senkung in der Injektionsposition des genannten Mechanismus verläuft.

## Claims

1. Injection device comprising a syringe body (1, 13), which delimits a chamber which is designed to be filled with a liquid, in particular a medicinal liquid, and a unit (17) for closing off the chamber and distributing the liquid, comprising an end piece (17a) for closing off the said chamber, which is extended by a locking male conical connection (17c), provided with a duct (18, 19) for distribution of the liquid,
- the said unit (17) for closing off and distributing being mobile axially between a position known as the closing-off position, in which the distribution duct (18, 19) is isolated from the chamber of the syringe body (1, 13), and a position known as the injection position, in which the said communication duct communicates with the said chamber,
- wherein the unit (17) for closing off and distributing comprises an intermediate collar (20), which is disposed between the closing-off end piece (17a) and the male conical connection (17c), and is placed such as to extend in an extension (3) of the syringe body (1, 13),
- wherein the syringe body (1, 13) comprises as extension a ring (3) which is provided with a front wall (10), in which there is provided an opening (11) for passage of the conical male connection (17c), the said ring delimiting two inner, upper (8) and lower (7) compartments which are juxtaposed axially, each of which has a shape which is conjugated with the intermediate collar (20) of the unit (17) for closing off and distributing, and can accommodate the said intermediate collar, the said compartments being separated by axial stop units (9) which can permit axial displacement of the intermediate collar (20) of one compartment (7, 8) towards the other compartment (8, 7),
wherein the said injection device is **characterised in that** the ring (3) has an extension in relation to the front wall (10), consisting of a sleeve (12) which is threaded internally, and is designed to accommodate the male conical connection (17c) and to act as a fixed nut for assembly of a locking female conical connection (30) which supports an injection needle (24).

2. Injection device according to claim 1, **characterised in that** the ring (3) has in its interior an annular neck (9) which forms the top of two longitudinal divergent sections (9a, 9b), and constitutes the axial stop means for separation of the upper (8) and lower (7) compartments, the intermediate collar (20) of the unit (17) for closing off and distributing having a shape and dimensions which are conjugated with the upper compartment (8) of the said ring.

3. Injection device according to claim 1 or claim 2, **characterised in that**:
- it comprises a closing-off stopper (13) with dimensions which are suitable for being inserted in the syringe body, provided with a through bore (15) which opens into the said syringe body at a countersink (16);
- the unit (17) for closing off and distributing has dimensions which are designed to be inserted in the bore (15) in the closing-off stopper (13), and comprises a distribution duct (18, 19) containing a transverse branch (19), which is disposed such as to be positioned recessed from the countersink (16) in the said closing-off stopper (13), in the closing-off position of the unit (17) for closing off and distributing, and to extend into the said countersink, in the injection position of the said unit.
